# EUROPEAN PATENT SPECIFICATION

(11) **EP 3 619 315 B1**
(45) Date of publication and mention of the grant of the patent: **30.09.2026**
(21) Application number: 18794468.1
(22) Date of filing: 04.05.2018
(51) Int. Cl.: C12P 7/06, C12P 7/14, A23J 3/20, A23K 10/16, A23K 10/12

(54) **SINGLE CELL PROTEIN PRODUCTS AND AN INTEGRATED METHOD FOR THE PRODUCTION OF ETHANOL AND SINGLE CELL PROTEIN**
EINZELZELLPROTEINPRODUKTE UND INTEGRIERTES VERFAHREN ZUR HERSTELLUNG VON ETHANOL UND EINZELZELLPROTEIN
PRODUITS PROTÉIQUES UNICELLULAIRES ET PROCÉDÉ INTÉGRÉ DE PRODUCTION D'ÉTHANOL ET DE PROTÉINE UNICELLULAIRE

(30) Priority: 05.05.2017 US 201762502073 P
(43) Date of publication of application: 11.03.2020
(73) Proprietor: Superbrewed Food, Inc., New Castle, Delaware 19720 (US)
(72) Inventor: TRACY, Bryan, P., Wilmington, DE 19806 (US); JONES, Shawn, William, Bear, DE 19701 (US); PHILLIPS, John, Randall, Middletown, DE 19709 (US); MITCHELL, Daniel, Knox, Wilmington, DE 19808 (US); EYAL, Aharon, M., 93629 Jerusalem (IL)
(74) Representative: Harrison IP Limited
(86) International application number: PCT/US2018/031101
(87) International publication number: WO 2018/204792

(56) References cited:
- WO-A1-2005/002606
- WO-A1-2016/153247
- WO-A1-2016/187494
- US-A- 4 439 525
- US-A- 5 985 336
- US-A1- 2005 163 802
- US-A1- 2007 141 083
- US-A1- 2009 291 469
- YOULING GAO: "Improved nutritional value of fish feed with plant protein ingredients by means of organic acid salts and solid state fermentation (Thesis)", 1 January 2011 (2011-01-01), Depart. of Animal and Aquacultural Sciences Norwegian University of Life Sciences, XP055557434, ISBN: 978-82-575-0987-3, Retrieved from the Internet <URL:https://brage.bibsys.no/xmlui/bitstream/handle/11250/2434102/2011-23_Youling%20Gao_%28IHA%29.pdf?sequence=1> [retrieved on 20190215]
- GAO: "Improved nutritional value of fish feed with plant protein ingredients by means of organic acid salts and solid state fermentation", DEPART. OF ANIMAL AND AQUACULTURAL SCIENCES NORWEGIAN UNIVERSITY OF LIFE SCIENCES, 2011, pages 1 - 24, XP055557434

## Description

### Field of the Invention

The field of art to which this invention generally pertains is Single cell protein products and more specifically to a single-cell protein product comprising 62%-75% by weight crude protein, at least 0.2% by weight butyric acid and at least three of (i) 5.5%-6.9% by weight lysine; (ii) 2.1%-3.5% by weight methionine; (iii) 3.1%-4.4% by weight threonine; (iv) 2.0%-3.3% by weight tryptophan and (v) 2.5%-4.5% by weight crude fat; and an integrated method for the production of ethanol and the single cell protein product.

### Background of the invention

Protein is an essential part of any diet. Increasing world population increases the demand for protein. Vegetable proteins are less attractive sources, since they are relatively poor in some essential amino acids, e.g. lysine, methionine, tryptophan and tyrosine. Animal sources provide the full range of essential amino acids needed by humans in sufficient quantities. The way in which protein is produced has significant impacts on both the environment and human health. Fish and seafood are a critical source of protein for 3 billion people, but the ocean's supply has reached its limit. Farmed fish is likely to make up the gap and is predicted to represent 2/3 of global supply by 2030. Fish is an efficient converter of feed to gained mass (a ratio of 1.2 compared to 6 for cows), but fish farming is fast reaching its limit as well. Farmed fish is heavily dependent on wild caught fish for feed (fishmeal), which provides protein and fish oil.

Additionally, increased population increases the need for ethanol as a green fuel. An integrated and synergistic production of ethanol with high value proteins sources is highly desired. Single-cell protein is such source of high-value proteins. Background art includes PCT Publication No. WO 2016/187494, which discloses animal feed comprising microbial biomass and methods of producing animal feed by culturing a microorganism to produce microbial biomass; PCT Publication No. WO 2005/002606, which discloses use of a single-cell protein material; and PCT Publication No. WO 2016/153247, which discloses Clostridium butyricum having immune enhancement and antiviral activities and use thereof.

### Summary of the invention

Provided is a single-cell protein product comprising on dry basis between 62% by weight and 75% by weight crude protein, at least 0.2% by weight butyric acid and at least three of, at least four of or all five of (i) between 5.5% by weight and 6.9% by weight lysine; (ii) between 2.1% by weight and 3.5% by weight methionine; (iii) between 3.1% by weight and 4.4% by weight threonine; (iv) between 2.0% by weight and 3.3% by weight tryptophan and (v) between 2.5% by weight and 4.5% by weight crude fat. According to an embodiment, the single-cell protein product, comprising on dry basis between 0.2% by weight and 2.0% by weight butyric acid. According to an embodiment, the single-cell protein product, comprising on dry basis between 4% by weight and 8% by weight ash. Further provided is the use of the single-cell protein product in an animal feed.

An integrated method for the production of ethanol and a single cell protein product is also provided, comprising providing a fermentation medium comprising a carbohydrate and undissolved protein; culturing yeast in the medium until less than 95% by weight of the fermentation medium carbohydrate is metabolized, whereby a fraction of the fermentation medium carbohydrate is metabolized and carbon dioxide and a fermentation liquor are generated, which fermentation liquor comprises ethanol, residual carbohydrate, undissolved protein and optionally byproduct glycerol; processing the fermentation liquor, which processing comprises distilling, separating solids and evaporating, whereby separated ethanol, a separated protein cake and a syrup are formed, which syrup comprises residual carbohydrate, undissolved protein and optionally byproduct glycerol; separating undissolved protein from the syrup, whereby separated undissolved protein and a sweet syrup are formed, which sweet syrup comprises residual carbohydrate and optionally byproduct glycerol; culturing a selected organism in the sweet syrup, whereby the residual carbohydrate and optionally at least one of the byproduct glycerol and carbon dioxide are metabolized and an SCP-comprising broth is formed; separating SCP from the SCP-comprising broth, whereby crude SCP and modified syrup are formed; optionally washing the crude SCP, whereby washed SCP is formed; and drying the crude SCP or the washed SCP; wherein the combined carbon content of the residual carbohydrate and the byproduct glycerol in the fermentation liquor is at least 3% of that in the fermentation medium carbohydrate; and the selected organism comprises an acetogenic organism.

According to an embodiment of provided method the culturing yeast specific duration is selected so that less than 95% by weight of the fermentation medium carbohydrate is metabolized. According to an embodiment of provided method the fermentation liquor comprises at least 10% ethanol. According to an embodiment of provided method the fermentation liquor comprises at least 0.5% glycerol. According to an embodiment of provided method the fermentation liquor residual carbohydrate comprises at least 0.5% oligosaccharide.

According to an embodiment of provided method the selected organism further comprises an organism capable of metabolizing glycerol, oligosaccharides or a combination thereof. According to an embodiment of provided method the culturing selected organism is anaerobic. According to an embodiment the provided method further comprising lysing the crude SCP, the washed SCP, the dried crude SCP and/or the dried washed SCP. According to an embodiment the provided method further comprises adding an exogenous carbon source to the syrup, sweet syrup or modified sweet syrup, wherein the exogenous carbon source is metabolized in the culturing selected organism.
According to an embodiment of provided method the biomass generation yield is at least 15% weight calculated on combined weight of carbohydrate and glycerol. According to an embodiment of provided method the biomass generation productivity is at least 0.5 g/L/hr.

### Detailed description of the invention

The particulars shown herein are by way of example and for purposes of illustrative discussion of the various embodiments of the present invention only and are presented in the cause of providing what is believed to be the most useful and readily understood description of the principles and conceptual aspects of the invention. In this regard, no attempt is made to show details of the invention in more detail than is necessary for a fundamental understanding of the invention, the description making apparent to those skilled in the art how the several forms of the invention may be embodied in practice.

The present invention will now be described by reference to more detailed embodiments. This invention may, however, be embodied in different forms and should not be construed as limited to the embodiments set forth herein. Rather, these embodiments are provided so that this disclosure will be thorough and complete, and will fully convey the scope of the invention to those skilled in the art.

Unless otherwise defined, all technical and scientific terms used herein have the same meaning as commonly understood by one of ordinary skill in the art to which this invention belongs. The terminology used in the description of the invention herein is for describing particular embodiments only and is not intended to be limiting of the invention. As used in the description of the invention and the appended claims, the singular forms "a," "an," and "the" are intended to include the plural forms as well, unless the context clearly indicates otherwise.

Unless otherwise indicated, all numbers expressing quantities of ingredients, reaction conditions, and so forth used in the specification and claims are to be understood as being modified in all instances by the term "about." Accordingly, unless indicated to the contrary, the numerical parameters set forth in the following specification and attached claims are approximations that may vary depending upon the desired properties sought to be obtained by the present invention. At the very least, and not as an attempt to limit the application of the doctrine of equivalents to the scope of the claims, each numerical parameter should be construed in light of the number of significant digits and ordinary rounding approaches.

Additional advantages of the invention will be set forth in part in the description which follows, and in part will be obvious from the description, or may be learned by practice of the invention. It is to be understood that both the foregoing general description and the following detailed description are exemplary and explanatory only and are not restrictive of the invention, as claimed

### As used herein SCP refers to Single-Cell Protein

Unless indicated otherwise, percent is weight percent and ratio is weight/weight ratio.

Provided is a single-cell protein product comprising on dry basis between 62% by weight and 75% by weight crude protein, between 64% and 71%, between 66% and 70%, at least 0.2% by weight butyric acid and at least three of, at least four of or all five of (i) between 5.5% by weight and 6.9% by weight lysine, between 5.9% and 6.5%, between 6.1% and 6.4%; (ii) between 2.1% by weight and 3.5% by weight methionine, between 2.5% and 3.3%, between 2.7% and 3%; (iii) between 3.1% by weight and 4.4% by weight threonine, between 3.4% and 4.2%, between 3.6% and 4%; (iv) between 2.0% by weight and 3.3% by weight tryptophan, between 2.3% and 3.1%, between 2.5% and 2.9%; and (v) between 2.5% by weight and 4.5% by weight crude fat between 2.8% and 4%, between 3% and 3.8%. According to an embodiment, the single-cell protein product comprising on dry basis between 0.2% by weight and 2.0% by weight butyric acid between 0.4% and 1.8%, between 0.5% and 1.5%. According to an embodiment, the single-cell protein product comprising on dry basis between 4% by weight and 8% by weight ash between 5% and 7%, between 5.5% and 6.5%. Further provided is use of the single-cell protein product in an animal feed.

Further provided is an integrated method for the production of ethanol and the single cell protein product comprising providing a fermentation medium comprising a carbohydrate and undissolved protein; culturing yeast in the medium until less than 95% by weight of the fermentation medium is metabolized, whereby a fraction of the fermentation medium carbohydrate is metabolized and carbon dioxide and a fermentation liquor are generated, which fermentation liquor comprises ethanol, residual carbohydrate, undissolved protein and optionally byproduct glycerol; processing the fermentation liquor, which processing comprises distilling, separating solids and evaporating, whereby separated ethanol, a separated protein cake and a syrup are formed, which syrup comprises residual carbohydrate, undissolved protein and optionally byproduct glycerol;
separating undissolved protein from the syrup, whereby separated undissolved protein and a sweet syrup are formed, which sweet syrup comprises residual carbohydrate and optionally byproduct glycerol; culturing a selected organism in the sweet syrup, whereby the residual carbohydrate and optionally at least one of the byproduct glycerol and carbon dioxide are metabolized and an SCP-comprising broth is formed; separating SCP from the SCP-comprising broth, whereby crude SCP and modified syrup are formed; optionally washing the crude SCP, whereby washed SCP is formed; and drying the crude or washed SCP; wherein the combined carbon content of the residual carbohydrate and the byproduct glycerol in the fermentation liquor is at least 3% of that in the fermentation medium carbohydrate; and the selected organism comprises an acetogenic organism.

Corn dry milling technology for ethanol production involves yeast fermentation of a hydrolyzate of corn starch, whereby a fermentation liquor is formed. That fermentation liquor contains produced ethanol, yeast biomass and residual corn kernel components, such as corn protein, corn oil and corn fiber, as well as additional fermentation coproducts. In a typical operation, ethanol is distilled out, leaving a stillage containing the other components, typically referred to as whole stillage. Next, insoluble components of the whole stillage (mainly insoluble protein and fiber) are filtered out to form stillage solids or cake and a filtrate named thin stillage. Typically, the thin stillage is concentrated up to form a concentrate, which is blended back with the stillage solids to form Dried Distillers Grains and Solubles (DDGS), which is a well-known and widely used feed ingredient. Whole stillage, thin stillage and DDGS typically contain glycerol and/or carbohydrates, which interfere with drying and formulation. In contrary, the modified syrup of the present invention is low on glycerol and/or on carbohydrate, making it an attractive feed ingredient as such and/or in combination with stillage solids to form an improved DDGS. Hence, further provided is DDGS wherein glycerol content on dry basis is less than 3%, less than 2%, less than 1%, or less than 0.5%. Additionally or alternatively, further provided is DDGS wherein carbohydrate content on dry basis is less than 3%, less than 2%, less than 1%, or less than 0.5%.

Any carbohydrate is suitable. According to an embodiment, the carbohydrate is a product of enzymatic hydrolysis of starch, e.g. corn starch, e.g. glucose, oligosaccharides and combinations thereof.

Any form of providing the fermentation medium is suitable. According to an embodiment, the providing comprises corn milling. According to an embodiment the providing comprises corn dry milling, optionally comprising starch gelatinization and enzymatic hydrolysis.

According to an embodiment the method comprises culturing yeast in the medium for a specific duration, whereby a fraction of the fermentation medium carbohydrate is metabolized and carbon dioxide and a fermentation liquor are generated, which fermentation liquor comprises ethanol, residual carbohydrate, and optionally byproduct glycerol.

According to current commercial practices, ethanol production is conducted at conditions wherein metabolizing of fermentation liquor carbohydrates approaches the theoretical yields, which in turn leads to nearly theoretical yields on ethanol production. This, however, comes at a cost in providing the fermentation medium, in metabolizing of the carbohydrates and/or in ethanol recovery from the formed fermentation liquor.

Thus, yeast may not metabolize oligosaccharides, so that optimizing metabolizing yields may require nearly complete hydrolysis of starch into glucose in providing the fermentation medium. Improving starch hydrolysis comes at the cost of increased enzyme consumptions and/or increased capital and energy cost in the hydrolysis step.

Higher fermentation yields can be reached by extending the duration of culturing, which comes at the cost of increased fermentation capital cost.

Additionally, at high fermentation medium glucose concentration, yeast tends to convert a fraction of the carbohydrate into glycerol, rather than to ethanol. Glucose concentration in the fermentation medium can be kept relatively low, but that results in lower concentration of ethanol in the formed fermentation liquor. One result is the need for larger fermenters, i.e. high fermentation capital yield, and higher capital and energy cost in recovering the formed ethanol.

Contrary to the current commercial practices, the parameters for the method of the present invention are selected to lead to lower costs of providing the medium, lower costs of fermenting the carbohydrates to ethanol and lower costs of ethanol recovery, even if compromising on the yield of fermenting carbohydrates to ethanol. Hence, according to an embodiment, the culturing yeast specific duration is selected so that less than 95% by weight of the fermentation medium carbohydrate is metabolized, less than 94%, less than 93%, less than 92%, less than 91%, less than 90%, less than 89%%, or less than 88%. According to an embodiment, the formed fermentation liquor comprises, in addition to ethanol, also residual carbohydrate and/or byproduct glycerol. According to an embodiment, the residual carbohydrate comprises an oligosaccharide selected from the group consisting of disaccharides, trisacchrides, higher molecular weight oligomers and combinations thereof. According to an embodiment, the combined carbon content of the residual carbohydrate and the byproduct glycerol in the fermentation liquor is at least 3% of that in the fermentation medium carbohydrate, at least 4%, at least 5%, at least 6%, at least 7%, at least 8%, at least 9%, at least 10%, at least 11%, or at least 12%. The carbon content can be calculated via the molecular formula, e.g. the carbon contents of a solution containing 12% glucose and one containing 2% glycerol are 12x72/180 and 2x36/92, respectively. According to an embodiment, the fermentation liquor comprises at least 10% ethanol, at least 12%, at least 14%, at least 16% or at least 17%. According to an embodiment the fermentation liquor comprises at least 0.5% glycerol, at least 0.6%, at least 0.7%, at least 0.8%, at least 0.9%, at least 1%, at least 1.1%, or at least 1.2%. Additionally or alternatively, according to an embodiment the fermentation liquor residual carbohydrate comprises at least 0.5% oligosaccharide, at least 0.6%, at least 0.7%, at least 0.8%, at least 0.9%, at least 1%, at least 1.1%, or at least 1.2%.

According to an embodiment the method comprises processing the fermentation liquor, which processing comprises distilling, separating solids and evaporating, whereby separated ethanol, a separated protein cake and a syrup are formed, which syrup comprises residual carbohydrate, undissolved protein and optionally byproduct glycerol. According to an embodiment, processing the fermentation liquor comprises (1) distilling ethanol to form separated ethanol and a whole stillage comprising residual carbohydrate, undissolved protein and optionally byproduct glycerol; (2) separating solids from the whole stillage, e.g. by filtration or centrifugation, whereby a separated protein cake and a thin stillage are formed and (3) evaporating water from the thin stillage, whereby a syrup is formed, which syrup comprises residual carbohydrate, optionally undissolved protein and optionally byproduct glycerol.

According to an embodiment the method comprises separating undissolved protein from the syrup, e.g. by filtration or centrifugation, whereby separated undissolved protein and a sweet syrup are formed, which sweet syrup comprises residual carbohydrate and optionally byproduct glycerol. According to an embodiment the method comprises washing the separated undissolved protein, whereby washed undissolved protein and a wash liquor are formed. As used herein, washing means washing with water or with a dilute aqueous process streams. Any form of washing is suitable. According to an embodiment the washing is repeated several times, in order to increase the efficiency of removing water-soluble compounds from the separated undissolved protein. According to an embodiment the wash liquor is combined with the sweet syrup, whereby a modified sweet syrup is formed;

According to an embodiment the method comprises culturing a selected organism in the syrup, in the sweet syrup or in the modified sweet syrup, whereby the residual carbohydrate and optionally at least one of the byproduct glycerol and carbon dioxide are metabolized and an SCP-comprising broth is formed. According to an embodiment, the selected organism comprises an acetogenic organism. According to an embodiment, the acetogenic organism is selected from the group consisting of *Butyribacterium methylotrophicum, Blautia product, Clostridium drakei, Clostridium magnum, Clostridium scatologenes, Clostridium ljungdahlii, Clostridium autoethanogenum, Clostridium aceticum, Clostriridum ragsdalei, Clostridium carboxidivorans, Eubacterium aggregans, Eubacterium limosum, Acetobacterium woodii, Oxobacter pfennigii, Treponema azotonutricium, Sporosoma ovata, Sporosoma termitida,* and *Terriporobacter glycolicus.* According to an embodiment, the selected organism further comprises an organism selected from the group consisting of organisms capable of metabolizing glycerol, oligosaccharides or a combination thereof. According to an embodiment, the organism capable of metabolizing glycerol, oligosaccharides or a combination thereof is selected from the group consisting of *Clostridium pasteurianum, Clostridium tyrobutyricum, Clostridium butyricum, Clostridium acetobutylicum, Clostridium beijerinckii, Clostridium sporogenes, Clostridium saccharoperbutylacetonicum,* and *Clostridium saccharobutylicum.*

According to an embodiment, the culturing selected organism is anaerobic.

According to an embodiment, the method comprises adding an exogenous carbon source to the syrup, sweet syrup or modified sweet syrup, wherein the exogenous carbon source is metabolized in the culturing selected organism.

According to an embodiment, biomass generation yield is at least 15% weight calculated on combined weight of carbohydrate and glycerol, at least 20%, at least 25%, at least 30% or at least 35%. Biomass generation yield is calculated according to the ratio between formed biomass on dry basis and the combined weight of carbohydrate and glycerol in the fermentation liquor and/or in the treated broth. According to an embodiment, biomass generation yield is at least 15% weight calculated on combined weight of carbohydrate and glycerol, at least 20%, at least 25%, at least 30% or at least 35%.

According to an embodiment, biomass generation productivity is at least 0.5 g/L/hr, at least 0.6 g/L/hr, at least 0.7 g/L/hr, at least 0.8 g/L/hr, at least 0.9 g/L/hr, or at least 1 g/L/hr.

According to an embodiment, the culturing selected organism generates, in addition to biomass, at least one metabolite selected from the group consisting of acetic acid, butyric acid, lactic acid, formic acid, 1,3-propanediol, n-butanol, ethanol, acetoin, and 2,3-butanediol. According to an embodiment, the concentration of butyric acid in the SCP-comprising broth is in the range between 0.5% and 15%.

According to an embodiment, the method comprises separating SCP from the SCP-comprising broth, whereby crude SCP and modified syrup are formed; According to an embodiment, the method comprises washing the crude SCP, whereby washed SCP is formed. According to an embodiment, the method comprises drying the SCP-comprising broth, the crude SCP or the washed SCP. According to an embodiment, the method comprises lysing the crude SCP, the washed SCP, the dried crude SCP and/or the dried washed SCP.

According to an embodiment, the dried crude SCP or the dried washed SCP, comprises on dry basis between 62% by weight and 75% by weight crude protein, between 64% and 74%, between 66% and 72%, at least 0.2% by weight butyric acid and at least three of, at least four of or all five of (i) between 5.5% by weight and 6.9% by weight lysine, between 5.9% and 6.6%, between 6.1% and 6.5%; (ii) between 2.1% by weight and 3.5% by weight methionine, between 2.5% and 3.3%, between 2.7% and 3%; (iii) between 3.1% by weight and 4.4% by weight threonine, between 3.2% and 4.3%, between 3.4% and 4%; (iv) between 2.0% by weight and 3.3% by weight tryptophan, between 2.5% and 3.3%, between 2.6% and 3%; and (v) between 2.5% by weight and 4.5% by weight crude fat between 2.6% and 4.4%, between 2.8% and 4%;. According to an embodiment the dried crude SCP or the dried washed SCP comprises on dry basis between 0.2% by weight and 2.0% by weight butyric acid. According to an embodiment the dried crude SCP or the dried washed SCP comprises on dry basis between 4% by weight and 8% by weight ash between 5% and 7%, between 5.5% and 6.5%.

Further provided is an integrated method for the production of ethanol and a single cell protein product, comprising (i) providing a fermentation medium comprising a carbohydrate and undissolved protein; (ii) culturing in the medium yeast and a selected organism for a specific duration, whereby the carbohydrate is metabolized and carbon dioxide and a fermentation liquor are generated, which fermentation liquor comprises ethanol and single cell protein; and (iii) processing the fermentation liquor, which processing comprises distilling, separating solids and drying, whereby separated ethanol and a protein product are formed; wherein (a) the culturing specific duration is selected so that the carbon content of the separated ethanol is less than 64% of the carbon content of the fermentation medium carbohydrate; (b) the culturing specific duration is selected so that the carbon content of the single cell protein is at least 3% of the carbon content of the fermentation medium carbohydrate; and (c) the selected organism comprises an acetogenic organism.

### Examples

### Example 1: Fermentation of syrup into SCP-comprising broth

Corn was fed into a standard dry-grind ethanol process. Specifically, the corn was milled, mixed into a slurry tank, and then liquified into a corn mash. The mash was then transferred to a fermentation tank with yeast for a simultaneous saccharification fermentation. This mash contained both carbohydrate and undissolved protein from the corn. During the fermentation, the yeast consumed the carbohydrate and produce primarily ethanol with glycerol as a minor product. The fermentation broth, or fermentation liquor, was then sent to distillation to remove the ethanol from the liquor and produce a separated ethanol. The remaining liquor at the bottom of the distillation columns was the whole stillage. The majority of the undissolved protein was removed from the whole stillage into a protein cake, and the remaining thin stillage was evaporated into a syrup. This syrup consisted of residual carbohydrate, residual undissolved protein, and glycerol.

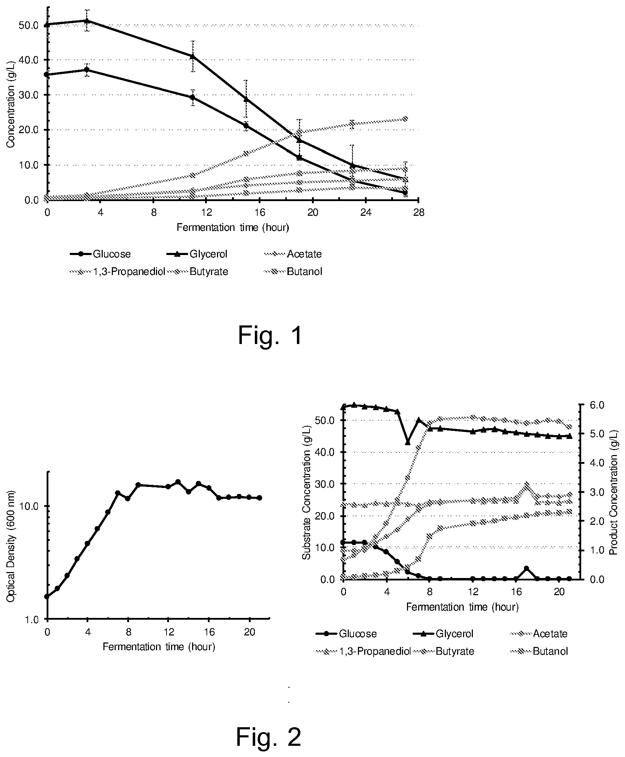

This syrup was diluted in a 1:1 ratio with sterile water and then 1.5 L of the diluted material was pumped into two replicate 3 L fermenters. Both fermenters were inoculated with a seed culture (20% v/v) of equal parts *Clostridium pasteurianum* and *Butyribacterium methylotrophicum,* an acetogenic bacteria. The fermenters were sparged with CO₂ at <25 mL/min, had minimal agitation (50-100 rpm), controlled at 37°C, and pH bottom controlled at 6.5 with 8M NaOH. Cell growth could not be monitored, as the culture was too turbid for an accurate optical density measurement, but the averaged metabolite profiles for the two fermenters are shown in Figure 1.

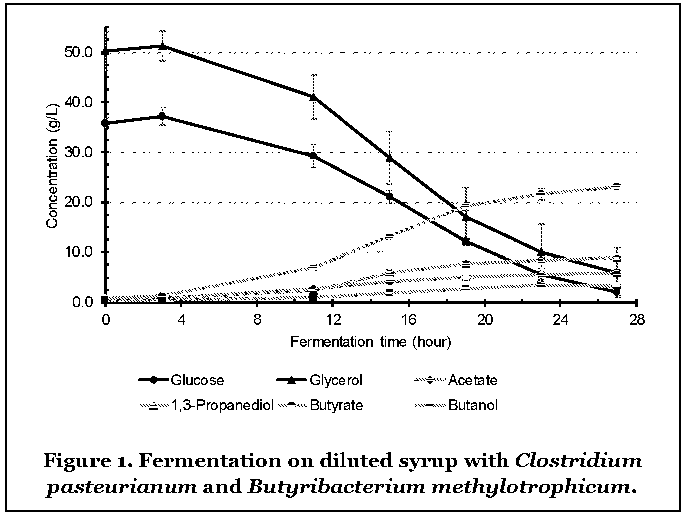

After 27 hours of growth, the culture consumed 33.6 g/L of glucose and 44.4 g/L of glycerol, while producing 22.4 g/L butyric acid, 8.1 g/L 1,3-propanediol, 5.3 g/L acetic acid, and 3.0 g/L n-butanol. The SCP-comprising broth was then dried and analyzed. Table 1 shows the proximate analysis of the dried material.

**Table 1. Proximate analysis of dried SCP-comprising broth.**

| **Characteristic** | **Dried SCP-comprising broth (average)** |
|---|---|
| Crude Protein | 31.1% (g/g) |
| Crude Fat | 8.6% (g/g) |
| Ash | 22.9% (g/g) |
| Butyric acid | 13.1% (g/g) |

| | |
|---|---|
| All values are on a dry weight basis. | |

### Example 2: Fermentation of sweet syrup into SCP-comprising broth

The raw syrup from Example 1 was processed through a microfiltration unit to remove the majority of the undissolved protein and other solids to generate a sweet syrup. About 5000 L of the sweet syrup was pumped into an 18000 L fermenter, and a trace elements and vitamin solutions were added to the syrup at 20 mL of solution per 1 L of syrup. The trace elements solution was 2 g/L nitrilotriacetic acid, 1 g/L MnSO₄·H₂O, 0.8 g/L Fe(SO₄)₂(NH₄)₂·6H₂O, 0.2 g/L CoCl₂·6H₂O, 0.2 mg/L ZnSO₄·6H₂O, 0.02 g/L CuCl₂·2H₂O, 0.02 g/L NiCl₂·6H₂O, 0.02 g/L Na₂MoO₄·2H₂O, 0.02 g/L Na₂SeO₄, and 0.02 g/L Na₂WO₄. The vitamin solution was 2 mg/L biotin, 2 mg/L folic acid, 10 mg/L pyridoxine-HCl, 5 mg/L thiamine-HCl, 5 mg/L riboflavin, 5 mg/L nicotinic acid, 5 mg/L calcium D-(+)-pantothenate, 0.1 mg/L vitamin b12, 5 mg/L p-aminobenzoic acid, and 5 mg/L thioctic acid. Additionally, antifoam was added at 0.1 mL/L.

The fermenter was inoculated with a seed culture (20% v/v) of equal parts Clostridium pasteurianum and Butyribacterium methylotrophicum, an acetogenic bacteria. The fermenters were sparged with CO₂ at <25 mL/min, controlled at 37°C, and pH bottom controlled at 6.5 with 8M NaOH. Cell growth was monitored along with metabolite profiles (Figure 2).

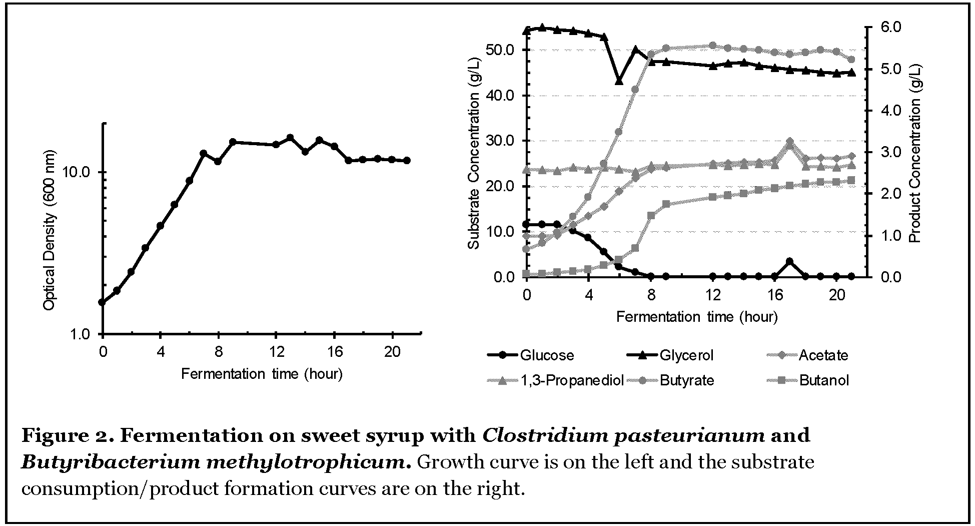

After 22 hours of growth, the culture reached a maximum optical density (OD) of 16.4 and produced 4.6 g/L butyric acid, 0.1 g/L 1,3-propanediol, 1.9 g/L acetic acid, and 2.2 g/L n-butanol. The SCP-comprising broth was then dried and analyzed. Table 2 shows the proximate analysis of the dried material. The dried cell mass was also acid hydrolyzed and run on an HPLC to determine the amino acid profile (Table 3).

**Table 2. Proximate analysis of dried SCP-comprising broth.**

| **Characteristic** | **Dried SCP-comprising broth** |
|---|---|
| Crude Protein | 17.3% (g/g) |
| Crude Fat | 2.2% (g/g) |
| Ash | 25.4% (g/g) |
| Butyric acid | 1.7% (g/g) |

| | |
|---|---|
| All values are on a dry weight basis. | |

**Table 3. Amino acid profile of dried SCP-comprising broth.**

| **Amino acid** | **Dried SCP-comprising broth** |
|---|---|
| Lysine | 0.45% (g/g) |
| Methionine | 0.10% (g/g) |
| Threonine | 0.33% (g/g) |
| Aspartic acid/asparagine | 3.35% (g/g) |
| Glutamic acid/glutamine | 6.31% (g/g) |
| Serine | 1.72% (g/g) |
| Histidine | 0.31% (g/g) |
| Glycine | 0.52% (g/g) |
| Arginine | 0.98% (g/g) |
| Alanine | 0.63% (g/g) |
| Tyrosine | 0.20% (g/g) |
| Valine | 0.34% (g/g) |
| Phenylalanine | 0.18% (g/g) |
| Isoleucine | 0.23% (g/g) |
| Leucine | 0.31% (g/g) |
| Proline | 0.33% (g/g) |

| | |
|---|---|
| All values are on a dry weight basis. | |

### Example 3: Fermentation of sweet syrup into crude SCP

The cells from the SCP-comprising broth in Example 2 were separated from the broth by centrifugation to generate a crude SCP. This crude SCP was dried and analyzed. Table 4 shows the proximate analysis of the dried material. The dried SCP was also acid hydrolyzed and run on an HPLC to determine the amino acid profile (Table 5).

**Table 4. Proximate analysis of crude SCP.**

| **Characteristic** | **Crude SCP** |
|---|---|
| Crude Protein | 45.9% (g/g) |
| Crude Fat | 1.1% (g/g) |
| Ash | 16.4% (g/g) |

| | |
|---|---|
| All values are on a dry weight basis. | |

**Table 3. Amino acid profile of crude SCP. Amino acid Crude SCP**

| **Amino acid** | **Crude SCP** |
|---|---|
| Lysine | 3.17% (g/g) |
| Methionine | 0.63% (g/g) |
| Threonine | 1.28% (g/g) |
| Aspartic acid/asparagine | 3.47% (g/g) |
| Glutamic acid/glutamine | 7.14% (g/g) |
| Serine | 1.88% (g/g) |
| Histidine | 0.98% (g/g) |
| Glycine | 2.84% (g/g) |
| Arginine | 3.98% (g/g) |
| Alanine | 3.00% (g/g) |
| Tyrosine | 1.04% (g/g) |
| Valine | 2.08% (g/g) |
| Phenylalanine | 1.19% (g/g) |
| Isoleucine | 1.68% (g/g) |
| Leucine | 2.47% (g/g) |
| Proline | 1.65% (g/g) |

| | |
|---|---|
| All values are on a dry weight basis. | |

### Example 4: Fermentation of sweet syrup into washed SCP

The crude SCP from Example 3 was washed at least once in water before being dried and analyzed. Table 5 shows the proximate analysis of the dried material. The dried SCP was also acid hydrolyzed and run on an HPLC to determine the amino acid profile (Table 6).

**Table 5. Proximate analysis of washed SCP.**

| **Characteristic** | **Washed SCP** |
|---|---|
| Crude Protein | 67.1% (g/g) |
| Crude Fat | 1.0% (g/g) |
| Ash | 13.0% (g/g) |

| | |
|---|---|
| All values are on a dry weight basis. | |

**Table 6. Amino acid profile of washed SCP.**

| **Amino acid** | **Washed SCP** |
|---|---|
| Lysine | 5.31% (g/g) |
| Methionine | 1.04% (g/g) |
| Threonine | 2.60% (g/g) |
| Aspartic acid/asparagine | 6.31% (g/g) |
| Glutamic acid/glutamine | 12.05% (g/g) |
| Serine | 2.85% (g/g) |
| Histidine | 1.49% (g/g) |
| Glycine | 3.73% (g/g) |
| Arginine | 4.45% (g/g) |
| Alanine | 4.73% (g/g) |
| Tyrosine | 1.77% (g/g) |
| Valine | 3.50% (g/g) |
| Phenylalanine | 2.18% (g/g) |
| Isoleucine | 3.17% (g/g) |
| Leucine | 4.31% (g/g) |
| Proline | 2.48% (g/g) |

| | |
|---|---|
| All values are on a dry weight basis. | |

### Example 5: Fermentation of modified sweet syrup into SCP-comprising broth

The raw syrup from Example 1 was processed through a microfiltration unit to remove the majority of the undissolved protein and other solids to generate a sweet syrup. The solids fraction from the microfiltration unit (primarily undissolved protein) was washed once with water, and the wash was mixed with the sweet syrup to generate a modified sweet syrup. About 5000 L of the modified sweet syrup was pumped into an 18000 L fermenter, and a trace elements and vitamin solutions were added to the modified syrup at 20 mL of solution per 1 L of syrup. The trace elements solution was 2 g/L nitrilotriacetic acid, 1 g/L MnSO₄·H₂O, 0.8 g/L Fe(SO₄)₂(NH₄)₂·6H₂O, 0.2 g/L CoCl₂·6H₂O, 0.2 mg/L ZnSO₄·6H₂O, 0.02 g/L CuCl₂·2H₂O, 0.02 g/L NiCl₂·6H₂O, 0.02 g/L Na₂MoO₄·2H₂O, 0.02 g/L Na₂SeO₄, and 0.02 g/L Na₂WO₄. The vitamin solution was 2 mg/L biotin, 2 mg/L folic acid, 10 mg/L pyridoxine-HCl, 5 mg/L thiamine-HCl, 5 mg/L riboflavin, 5 mg/L nicotinic acid, 5 mg/L calcium D-(+)-pantothenate, 0.1 mg/L vitamin b12, 5 mg/L p-aminobenzoic acid, and 5 mg/L thioctic acid. Additionally, antifoam was added at 0.1 mL/L.

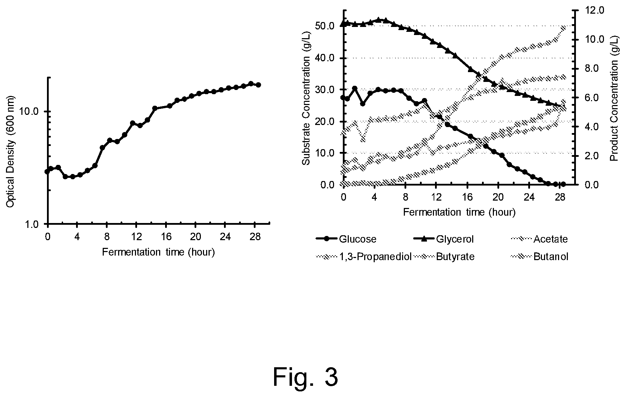

The fermenter was inoculated with a seed culture (20% v/v) of equal parts *Clostridium pasteurianum* and *Butyribacterium methylotrophicum,* an acetogenic bacteria. The fermenters were sparged with CO₂ at <25 mL/min, controlled at 37°C, and pH bottom controlled at 6.5 with 8M NaOH. Cell growth was monitored along with metabolite profiles (Figure 3).

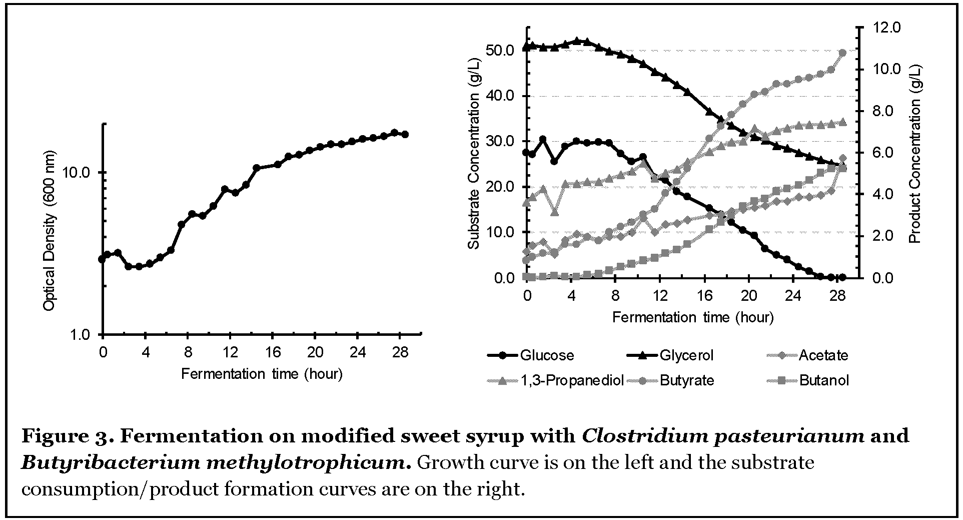

After 29 hours of growth, the culture reached a maximum optical density (OD) of 17.7 and produced 9.9 g/L butyric acid, 3.8 g/L 1,3-propanediol, 4.5 g/L acetic acid, and 5.2 g/L n-butanol. The SCP-comprising broth was then dried and analyzed. Table 7 shows the proximate analysis of the dried material. The dried cell mass was also acid hydrolyzed and run on an HPLC to determine the amino acid profile (Table 8).

**Table 7. Proximate analysis of SCP-comprising broth.**

| **Characteristic** | **Dried SCP-comprising broth** |
|---|---|
| Crude Protein | 15.7% (g/g) |
| Crude Fat | 0.7% (g/g) |
| Ash | 26.6% (g/g) |

| | |
|---|---|
| All values are on a dry weight basis. | |

**Table 8. Amino acid profile of SCP-comprising broth.**

| **Amino acid** | **Dried SCP-comprising broth** |
|---|---|
| Lysine | 0.92% (g/g) |
| Methionine | 0.23% (g/g) |
| Threonine | 0.83% (g/g) |
| Aspartic acid/asparagine | 1.60% (g/g) |
| Glutamic acid/glutamine | 2.89% (g/g) |
| Serine | 0.82% (g/g) |
| Histidine | 0.59% (g/g) |
| Glycine | 1.11% (g/g) |
| Arginine | 0.82% (g/g) |
| Alanine | 1.53% (g/g) |
| Tyrosine | 0.44% (g/g) |
| Valine | 0.90% (g/g) |
| Phenylalanine | 0.47% (g/g) |
| Isoleucine | 0.59% (g/g) |
| Leucine | 0.79% (g/g) |
| Proline | 0.66% (g/g) |

| | |
|---|---|
| All values are on a dry weight basis. | |

### Example 6: Fermentation of modified sweet syrup into washed SCP

The cells from the SCP-comprising broth in Example 5 were separated from the broth by centrifugation to generate a crude SCP. This crude SCP was washed at least once in water before being dried and analyzed. Table 9 shows the proximate analysis of the dried material. The dried SCP was also acid hydrolyzed and run on an HPLC to determine the amino acid profile (Table 10).

**Table 9. Proximate analysis of washed SCP.**

| **Characteristic** | **Washed SCP** |
|---|---|
| Crude Protein | 61.7% (g/g) |
| Crude Fat | 1.5% (g/g) |
| Ash | 13.8% (g/g) |

| | |
|---|---|
| All values are on a dry weight basis. | |

**Table 10. Amino acid profile of washed SCP.**

| **Amino acid** | **Washed SCP** |
|---|---|
| Lysine | 5.34% (g/g) |
| Methionine | 1.96% (g/g) |
| Threonine | 3.19% (g/g) |
| Aspartic acid/asparagine | 6.46% (g/g) |
| Glutamic acid/glutamine | 11.46% (g/g) |
| Serine | 2.83% (g/g) |
| Histidine | 1.90% (g/g) |
| Glycine | 3.59% (g/g) |
| Arginine | 3.51% (g/g) |
| Alanine | 4.59% (g/g) |
| Tyrosine | 1.29% (g/g) |
| Valine | 3.08% (g/g) |
| Phenylalanine | 2.05% (g/g) |
| Isoleucine | 2.93% (g/g) |
| Leucine | 3.99% (g/g) |
| Proline | 3.54% (g/g) |

| | |
|---|---|
| All values are on a dry weight basis. | |

## Claims

1. A single-cell protein product comprising on dry basis between 62% by weight and 75% by weight crude protein, at least 0.2% by weight butyric acid and at least three of, at least four of or all five of (i) between 5.5% by weight and 6.9% by weight lysine; (ii) between 2.1% by weight and 3.5% by weight methionine; (iii) between 3.1% by weight and 4.4% by weight threonine; (iv) between 2.0% by weight and 3.3% by weight tryptophan and (v) between 2.5% by weight and 4.5% by weight crude fat.

2. The single-cell protein product of Claim 1, comprising on dry basis between 0.2% by weight and 2.0% by weight butyric acid.

3. The single-cell protein product of Claim 1 or Claim 2, comprising on dry basis between 4% by weight and 8% by weight ash.

4. Use of the single-cell protein product of any one of Claims 1 to 3 in an animal feed.

5. An integrated method for the production of ethanol and said single cell protein product of any one of Claims 1 to 3, comprising
(i) providing a fermentation medium comprising a carbohydrate and undissolved protein;
(ii) culturing yeast in said medium until less than 95% by weight of said fermentation medium carbohydrate is metabolized , whereby a fraction of said fermentation medium carbohydrate is metabolized and carbon dioxide and a fermentation liquor are generated, which fermentation liquor comprises ethanol, residual carbohydrate, undissolved protein and optionally byproduct glycerol;
(iii) processing said fermentation liquor, which processing comprises distilling, separating solids and evaporating, whereby separated ethanol, a separated protein cake and a syrup are formed, which syrup comprises residual carbohydrate, undissolved protein and optionally byproduct glycerol;
(iv) separating undissolved protein from said syrup, whereby separated undissolved protein and a sweet syrup are formed, which sweet syrup comprises residual carbohydrate and optionally byproduct glycerol;
(v) culturing a selected organism in said sweet syrup, whereby said residual carbohydrate and optionally at least one of said byproduct glycerol and carbon dioxide are metabolized and an SCP-comprising broth is formed;
(vi) separating SCP from said SCP-comprising broth, whereby crude SCP and modified syrup are formed;
(vii) optionally washing said crude SCP, whereby washed SCP is formed; and
(viii) drying said crude or said washed SCP;
wherein
(a) the combined carbon content of said residual carbohydrate and said byproduct glycerol, when present, in said fermentation liquor of step (ii) is at least 3% of that in the fermentation medium carbohydrate; and
(b) said selected organism comprises an acetogenic organism.

6. The method of Claim 5, wherein said fermentation liquor comprises at least 10% ethanol.

7. The method of any one of Claims 5 to 6, wherein said fermentation liquor comprises at least 0.5% glycerol.

8. The method of any one of Claims 5 to 7, wherein said fermentation liquor residual carbohydrate comprises at least 0.5% oligosaccharide.

9. The method of any one of Claims 5 to 8, wherein said selected organism further comprises an organism capable of metabolizing glycerol, oligosaccharides or a combination thereof.

10. The method of any one of Claims 5 to 9, wherein said culturing selected organism is anaerobic.

11. The method of any one of Claims 5 to 10, further comprising lysing said crude SCP, said washed SCP, said dried crude SCP and/or said dried washed SCP.

12. The method of any one of Claims 5 to 11, further comprising adding an exogenous carbon source to at least one selected from the group consisting of said syrup of step (iii), sweet syrup of step (iv) or modified syrup of step (vi), wherein said exogenous carbon source is metabolized in said culturing selected organism.

13. The method of any one of Claims 5 to 12, wherein biomass generation yield is at least 15% weight calculated on combined weight of carbohydrate and glycerol.

14. The method of any one of Claims 5 to 13, wherein biomass generation productivity is at least 0.5 g/L/hr.

## Patentansprüche

1. Einzelzellproteinprodukt, umfassend, auf Trockenbasis, zwischen 62 Gewichts-% und 75 Gewichts-% Rohprotein, mindestens 0,2 Gewichts-% Buttersäure und mindestens drei von, mindestens vier von oder alle fünf von (i) zwischen 5,5 Gewichts-% und 6,9 Gewichts-% Lysin; (ii) zwischen 2,1 Gewichts-% und 3,5 Gewichts-% Methionin; (iii) zwischen 3,1 Gewichts-% und 4,4 Gewichts-% Threonin; (iv) zwischen 2,0 Gewichts-% und 3,3 Gewichts-% Tryptophan und (v) zwischen 2,5 Gewichts-% und 4,5 Gewichts-% Rohfett.

2. Einzelzellproteinprodukt nach Anspruch 1, umfassend, auf Trockenbasis, zwischen 0,2 Gewichts-% und 2,0 Gewichts-% Buttersäure.

3. Einzelzellproteinprodukt nach Anspruch 1 oder Anspruch 2, umfassend, auf Trockenbasis, zwischen 4 Gewichts-% und 8 Gewichts-% Asche.

4. Verwendung des Einzelzellproteinprodukts nach einem der Ansprüche 1 bis 3 in einem Tierfutter.

5. Integriertes Verfahren zur Herstellung von Ethanol und des Einzelzellproteinprodukts nach einem der Ansprüche 1 bis 3, umfassend
(i) Bereitstellen eines Fermentationsmediums, das ein Kohlenhydrat und ungelöstes Protein umfasst;
(ii) Kultivieren von Hefe in dem Medium, bis weniger als 95 Gewichts-% des Fermentationsmedium-Kohlenhydrats metabolisiert sind, wodurch eine Fraktion des Fermentationsmedium-Kohlenhydrats metabolisiert wird und Kohlendioxid und eine Fermentationsflüssigkeit erzeugt werden, wobei die Fermentationsflüssigkeit Ethanol, Restkohlenhydrat, ungelöstes Protein und optional das Nebenprodukt Glycerin umfasst;
(iii) Verarbeiten der Fermentationsflüssigkeit, wobei das Verarbeiten das Destillieren, Abtrennen von Feststoffen und Verdampfen umfasst, wodurch abgetrenntes Ethanol, ein abgetrennter Proteinkuchen und ein Sirup gebildet werden, wobei dieser Sirup Restkohlenhydrat, ungelöstes Protein und optional das Nebenprodukt Glycerin umfasst;
(iv) Abtrennen von ungelöstem Protein aus dem Sirup, wodurch abgetrenntes ungelöstes Protein und ein süßer Sirup gebildet werden, wobei der süße Sirup Restkohlenhydrat und optional das Nebenprodukt Glycerin umfasst;
(v) Kultivieren eines ausgewählten Organismus in dem süßen Sirup, wodurch das Restkohlenhydrat und optional mindestens eines des Nebenprodukts Glycerin und Kohlendioxids metabolisiert werden und eine SCP umfassende Brühe entsteht;
(vi) Abtrennen von SCP aus der SCP umfassenden Brühe, wodurch rohes SCP und modifizierter Sirup entstehen;
(vii) optional Waschen des rohen SCP, wodurch gewaschenes SCP entsteht; und
(viii) Trocknen des Rohprodukts oder des gewaschenen SCP;
wobei
(a) der kombinierte Kohlenstoffgehalt des Restkohlenhydrats und des Nebenprodukts Glycerin, falls vorhanden, in der Fermentationsbrühe von Schritt (ii) mindestens 3 % von demjenigen in dem Fermentationsmedium-Kohlenhydrat beträgt; und
(b) der ausgewählte Organismus einen acetogenen Organismus umfasst.

6. Verfahren nach Anspruch 5, wobei die Fermentationsflüssigkeit mindestens 10 % Ethanol umfasst.

7. Verfahren nach einem der Ansprüche 5 bis 6, wobei die Fermentationsflüssigkeit mindestens 0,5 % Glycerin umfasst.

8. Verfahren nach einem der Ansprüche 5 bis 7, wobei das Fermentationsflüssigkeits-Restkohlenhydrat mindestens 0,5 % Oligosaccharid umfasst.

9. Verfahren nach einem der Ansprüche 5 bis 8, wobei der ausgewählte Organismus ferner einen Organismus umfasst, der imstande ist, Glycerin, Oligosaccharide oder eine Kombination davon zu metabolisieren.

10. Verfahren nach einem der Ansprüche 5 bis 9, wobei der kultivierende ausgewählte Organismus anaerob ist.

11. Verfahren nach einem der Ansprüche 5 bis 10, ferner umfassend das Lysieren des rohen SCP, des gewaschenen SCP, des getrockneten rohen SCP und/oder des getrockneten gewaschenen SCP.

12. Verfahren nach einem der Ansprüche 5 bis 11, ferner umfassend das Hinzufügen einer exogenen Kohlenstoffquelle zu mindestens einem, ausgewählt aus der Gruppe bestehend aus dem Sirup von Schritt (iii), süßen Sirup von Schritt (iv) oder modifizierten Sirup von Schritt (vi), wobei die exogene Kohlenstoffquelle in dem kultivierenden ausgewählten Organismus metabolisiert wird.

13. Verfahren nach einem der Ansprüche 5 bis 12, wobei die Ausbeute der Biomasseerzeugung mindestens 15 % Gewicht, berechnet anhand des kombinierten Gewichts von Kohlenhydrat und Glycerin, beträgt.

14. Verfahren nach einem der Ansprüche 5 bis 13, wobei die Produktivität der Biomasseerzeugung mindestens 0,5 g/l/Std. beträgt.

## Revendications

1. Produit protéique unicellulaire comprenant sur une base sèche entre 62 % en poids et 75 % en poids de protéine brute, au moins 0,2 % en poids d'acide butyrique et au moins trois, au moins quatre ou la totalité des cinq parmi (i) entre 5,5 % en poids et 6,9 % en poids de lysine ; (ii) entre 2,1 % en poids et 3,5 % en poids de méthionine ; (iii) entre 3,1 % en poids et 4,4 % en poids de thréonine ; (iv) entre 2,0 % en poids et 3,3 % en poids de tryptophane et (v) entre 2,5 % en poids et 4,5 % en poids de matière grasse brute.

2. Produit protéique unicellulaire de la revendication 1, comprenant, sur une base sèche, entre 0,2 % en poids et 2,0 % en poids d'acide butyrique.

3. Produit protéique unicellulaire de la revendication 1 ou la revendication 2, comprenant sur une base sèche entre 4 % en poids et 8 % en poids de cendres.

4. Utilisation du produit protéique unicellulaire de l'une quelconque des revendications 1 à 3 dans un aliment pour animaux.

5. Procédé intégré permettant la production d'éthanol et dudit produit protéique unicellulaire de l'une quelconque des revendications 1 à 3, comprenant
(i) la fourniture d'un milieu de fermentation comprenant un glucide et une protéine non dissoute ;
(ii) la culture de levure dans ledit milieu jusqu'à ce que moins de 95 % en poids dudit glucide de milieu de fermentation soit métabolisé, moyennant quoi une fraction dudit glucide de milieu de fermentation est métabolisée et du dioxyde de carbone et une liqueur de fermentation sont générés, laquelle liqueur de fermentation comprend de l'éthanol, un glucide résiduel, une protéine non dissoute et éventuellement du glycérol de sous-produit ;
(iii) le traitement de ladite liqueur de fermentation, lequel traitement comprend la distillation, la séparation des solides et l'évaporation, moyennant quoi de l'éthanol séparé, un gâteau protéique séparé et un sirop sont formés, lequel sirop comprend un glucide résiduel, une protéine non dissoute et éventuellement du glycérol de sous-produit ;
(iv) la séparation de protéine non dissoute dudit sirop, moyennant quoi une protéine non dissoute séparée et un sirop sucré sont formés, lequel sirop sucré comprend un glucide résiduel et éventuellement du glycérol de sous-produit ;
(v) la culture d'un organisme choisi dans ledit sirop sucré, moyennant quoi ledit glucide résiduel et éventuellement au moins l'un dudit glycérol de sous-produit et dudit dioxyde de carbone sont métabolisés et un bouillon comprenant une SCP est formé ;
(vi) la séparation de SCP dudit bouillon comprenant une SCP, moyennant quoi une SCP brute et un sirop modifié sont formés ;
(vii) éventuellement, le lavage de ladite SCP brute, moyennant quoi une SCP lavée est formée ; et
(viii) le séchage de ladite SCP brute ou de ladite SCP lavée ;
dans lequel
(a) la teneur combinée en carbone dudit glucide résiduel et dudit glycérol de sous-produit, lorsqu'il est présent, dans ladite liqueur de fermentation de l'étape (ii) est d'au moins 3 % de celle du glucide de milieu de fermentation ; et
(b) ledit organisme choisi comprend un organisme acétogène.

6. Procédé de la revendication 5, dans lequel ladite liqueur de fermentation comprend au moins 10 % d'éthanol.

7. Procédé de l'une quelconque des revendications 5 à 6, dans lequel ladite liqueur de fermentation comprend au moins 0,5 % de glycérol.

8. Procédé de l'une quelconque des revendications 5 à 7, dans lequel ledit glucide résiduel de liqueur de fermentation comprend au moins 0,5 % d'oligosaccharide.

9. Procédé de l'une quelconque des revendications 5 à 8, dans lequel ledit organisme choisi comprend en outre un organisme capable de métaboliser le glycérol, les oligosaccharides ou une combinaison de ceux-ci.

10. Procédé de l'une quelconque des revendications 5 à 9, dans lequel ladite culture de l'organisme choisi est anaérobie.

11. Procédé de l'une quelconque des revendications 5 à 10, comprenant en outre la lyse de ladite SCP brute, de ladite SCP lavée, de ladite SCP brute séchée et/ou de ladite SCP lavée séchée.

12. Procédé de l'une quelconque des revendications 5 à 11, comprenant en outre l'ajout d'une source de carbone exogène à au moins l'un choisi dans le groupe constitué dudit sirop de l'étape (iii), dudit sirop sucré de l'étape (iv) ou dudit sirop modifié de l'étape (vi), dans lequel ladite source de carbone exogène est métabolisée lors de ladite culture de l'organisme choisi.

13. Procédé de l'une quelconque des revendications 5 à 12, dans lequel le rendement de génération de biomasse est d'au moins 15 % en poids calculé par rapport au poids combiné de glucide et de glycérol.

14. Procédé de l'une quelconque des revendications 5 à 13, dans lequel la productivité de génération de biomasse est d'au moins 0,5 g/l/h.
